# EUROPEAN PATENT APPLICATION

(11) **EP 1 172 143 A1**
(43) Date of publication of application: **16.01.2002**
(21) Application number: 00202509.6
(22) Date of filing: 14.07.2000
(51) Int. Cl.: B01J 31/40, C07C 45/80, B01J 38/56, B01J 38/60

(54) **Process for the recovery of rhodium**

(71) Applicant: DSM N.V., 6411 TE Heerlen (NL)
(72) Inventor: Gelling, Onko Jan, 6129 HV Stein (NL); Toth, Imre, 6166 GM Geleen (NL)
(74) Representative: Verhaegen, Ilse Maria M.

(57) **Abstract**

A process for the recovery of rhodium from an organic reaction mixture containing rhodium/phosphite ligand complex, phosphite degradation products and high-boiler organic compounds, wherein
A) the organic reaction mixture is subjected to an aqueous oxidative and/or acidic treatment, resulting in an aqueous-polar organic two-phase mixture,
B) the aqueous phase is removed,
C) solution of lipophilic phosphine and/or lipophilic phosphite in a non-polar solvent is added to the organic phase, which solvent is immiscible with the polar organic phase, resulting in a two-phase mixture containing a non-polar organic phase rich in rhodium and lipophilic phosphine and/or lipophilic phosphite and a polar organic phase rich in phosphite degradation products and high-boiler organic compounds, or the polar organic phase is extracted with an immiscible non-polar organic solvent, resulting in a two-phase mixture containing a non-polar organic phase rich in phosphite degradation products and a polar organic phase rich in rhodium,
D) the non-polar organic phase is separated from the polar organic phase.

## Description

This invention relates to a process for the recovery of rhodium from an organic reaction mixture containing rhodium/phosphite ligand complex, phosphite degradation products and high-boiler organic compounds. Furthermore, this invention also relates to the regeneration and recycling of the thus separated Rh as Rh/phosphite ligand complex.

Such a mixture is obtained in a process as described in WO-A-9906345. This publication discloses a process for the preparation of an aldehyde by hydroformylating an ethylenically unsaturated compound using a catalytic system composed of rhodium and a lipophilic bidentate phosphite ligand. The benefit of using such lipophilic bidentate phosphite ligand for the hydroformylation process is that the lipophilic catalyst can be readily separated from the generally non-lipophilic high-boiler compounds by extracting the catalytic system into a non-polar solvent like hexane. Subsequently, the catalyst can be recycled to the hydroformylation reactor either in the non-polar solution or after adding the process solvent and removing the non-polar solvent by evaporation.

A disadvantage of this process is that the continuously formed ligand degradation products (formed for example via hydrolysis, oxidation and/or thermal degradation) are for a substantial part co-extracted into the non-polar phase and hence are not separated from the catalyst system. This leads to an undesirable continuous build-up of ligand degradation products in the reactor zone.

It is an object of the present invention to provide an improved and satisfactory process for the separation and removal of high-boiler organic compounds and phosphite degradation products in combination with the recovery of rhodium from organic reaction mixtures containing rhodium/phosphite ligand complex, phosphite degradation products and high-boiler organic compounds. Furthermore, it is the object of the present invention to provide a method for the regeneration and recycling of such separated Rh-containing fraction as Rh/phosphite ligand complex.

The first object is achieved in that
A) the organic reaction mixture is subjected to an aqueous oxidative and/or acidic treatment, resulting in an aqueous-polar organic two-phase mixture,
B) the aqueous phase is removed,
C) a solution of lipophilic phosphine and/or lipophilic phosphite in a non-polar solvent is added to the organic phase, which solvent is immiscible with the polar organic phase, resulting in a two-phase mixture containing a non-polar organic phase rich in rhodium and lipophilic phosphine and/or lipophilic phosphite and a polar organic phase rich in phosphite degradation products and high-boiler organic compounds, or the polar organic phase is extracted with an immiscible non-polar organic solvent, resulting in a two-phase mixture containing a non-polar organic phase rich in phosphite degradation products and a polar organic phase rich in rhodium,
D) the non-polar organic phase is separated from the polar organic phase.

It has been found that the ligand degradation products and high-boilers can substantially be separated from an organic mixture also containing rhodium/phosphite ligand complex and rhodium can be subsequently recovered for reuse. An advantage of the present invention is that there is no or less accumulation of ligand degradation products and high-boilers in the hydroformylation reaction zone. This is advantageous because the effective reactor volume and productivity are not decreased. A further advantage of the process of the present invention is that the activity and/or selectivity of the catalyst system are maintained over a longer period of time. This is because ligand degradation compounds tend to gradually deactivate the catalyst. A prolonged period of high catalytic activity and/or selectivity is necessary for performing a continuous process. An additional advantage of the present invention is that it can be used in combination with the aldehyde/catalyst separation processes described in WO-A-9634687 and WO-A-9906345 resulting in an improved process for the separation of high-boiler compounds and phosphite degradation products from catalyst recycle streams.

The object of the present invention to provide a method for the recovery of rhodium from an organic reaction mixture containing rhodium/non-lipophilic phosphite ligand complex, non-lipophilic phosphite ligand degradation products and high-boiler organic compounds, regeneration and recycling of the separated Rh-containing fraction as Rh/non-lipophilic phosphite ligand complex is achieved in that
A) the organic reaction mixture is subjected to an aqueous oxidative and/or acidic treatment, resulting in an aqueous-polar organic two-phase mixture,
B) the aqueous phase is removed,
C) a solution of lipophilic phosphine and/or lipophilic phosphite in a non-polar solvent is added to the organic phase, which solvent is immiscible with the polar organic phase, resulting in a non-polar organic and polar organic two-phase mixture,
D) the non-polar organic phase rich in rhodium and lipophilic phosphine and/or lipophilic phosphite is separated from the polar organic phase rich in phosphite degradation products and high-boiler organic compounds,
E) a non-lipophilic phosphite ligand is added to the non-polar organic phase in a polar solution which is immiscible with the non-polar organic phase, resulting in a non-polar organic and polar two-phase mixture,
F) the non-polar organic phase rich in lipophilic phosphine and/or lipophilic phosphite is separated from the polar organic phase rich in rhodium and non-lipophilic phosphite ligand complex and
G) the polar organic phase is recycled to the reactor.

The object of the present invention to provide a method for the recovery of rhodium from an organic reaction mixture containing rhodium/lipophilic phosphite ligand complex, lipophilic phosphite ligand degradation products and high-boiler organic compounds, regeneration and recycling of the separated Rh-containing fraction as Rh/lipophilic phosphite ligand complex is achieved in that
A) the organic reaction mixture is subjected to an aqueous oxidative and/or acidic treatment, resulting in an aqueous-polar organic two-phase mixture,
B) the aqueous phase is removed,
C) the polar organic phase is extracted with an immiscible non-polar organic solvent, resulting in a two-phase mixture containing a non-polar organic phase rich in lipophilic phosphite degradation products and a polar organic phase rich in rhodium,
D) the non-polar organic phase is separated from the polar organic phase,
E) a solution of lipophilic phosphite in a non-polar solvent is added to the polar organic phase, which solvent is immiscible with the polar organic phase, resulting in a polar and non-polar organic two-phase mixture,
F) the non-polar organic phase containing rhodium/lipophilic phosphite ligand complex is separated from the polar organic phase, and
G) the non-polar organic phase is recycled to the reactor.

Since Rh is a very expensive metal, a highly efficient catalyst recycling is indispensable to the profitability of Rh-based commercial catalytic processes. For this reason the recovery and recycling of Rh from various process-streams are frequently addressed in the literature. For example, in commercial hydroformylation processes utilizing Rh in the presence of organophosphorous compounds as ligands in an organic reaction medium, the Rh-based catalyst is efficiently separated from the products and recycled by a distillation process. The Rh recycle procedure herein generally involves withdrawing a portion of the organic reaction medium containing the catalyst and aldehyde products from the hydroformylation zone, either continuously or intermittently, and distilling the aldehyde products and other volatile materials therefrom in one or more stages under atmospheric, reduced or elevated pressure, as appropriate, in a separate distillation zone. The Rh catalyst remains non-volatilized in the distillation residue of the said distillation procedure. Therefore, the Rh catalyst can be conveniently recycled to the hydroformylation reaction zone Rh with the distillation residue. It is well known in the art that during the hydroformylation of olefinic compounds, high-boiler organic compounds such as aldols, polymers, etc. are formed as byproducts beside the aldehyde products of the reaction. Therefore, the recycling of the Rh catalyst results in a build-up of high-boiler compounds in the hydroformylation reaction zone. The build-up of high-boilers is disadvantageous for the hydroformylation process as it decreases the effective reactor volume and productivity. In order to circumvent this problem the high-boiler products should be separated from the Rh-catalyst recycle stream and removed at some stage of the process. For this purpose, generally, a portion of the distillation residue containing the Rh catalyst is withdrawn continuously or alternatively the whole or a portion of the distillation residue is withdrawn periodically from the catalyst recycle stream as high-boiler purge. The withdrawal of such a secondary stream from the primary catalyst recycle might also be required in order to regenerate the catalyst when catalyst deactivation occurs during prolonged use due to decomposition or catalyst poising. An efficient separation of Rh from this secondary process stream, as well as the regeneration and recycling of Rh-organophosphorous catalyst are also essential to the economy of the process. The separation of Rh from high-boiler purges can be achieved by various means such as adsorption, extraction, membrane filtration, etc., which are well known in the art. The regeneration of Rh catalyst of such Rh-containing fractions is also well documented in the literature when the organophosphorous ligand is a mono-, bi- or multidentate phosphine derivative as for example described in EP-A-829300.

As described above, methods for recovering Rh from hydroformylation reaction mixtures catalyzed by Rh organophosphorous systems, including high-boiler purges therefrom, for reuse are known. However, none of them are fully satisfactory for industrial use when the organophosphorous ligand is an organobisphosphite compound. It is well known in the art that organobisphosphites may be employed as ligands for Rh based hydroformylation catalysts and that such catalysts exhibits exceptional activity and regioselectivity for producing aldehydes via olefin hydroformylation. For instance, US-A-4,668,651 and US-A-4,769,498 describe such hydroformylation. It is also known, that in contrast to organophosphines or organobisphosphines, organophosphites are highly sensitive to hydrolysis by traces of water and acids resulting in the formation of ligand degradation products. Hydrolysis of organobisphosphites results in the formation of for example phosphorous acid and monodentate phosphite derivatives like for example phosphite oxides. Organophosphites are also sensitive to degradation by alcoholysis in reaction with traces of alcohols, which are available, for example, by aldol dimerization or hydrogenation of product aldehydes. Details of such decompositions are for example described in US-A-5288918. Therefore, the chemical stability and decomposition pattern of a Rh-organophosphite based catalyst is different from that of Rh-organophosphine/bisphosphine based catalyst. In a commercial hydroformylation process based on Rh-organobisphosphites, not only the high-boiler byproducts of the hydroformylation reaction, but also the ligand degradation products should be removed from the high-boiler purge (secondary catalyst recycle stream) in order to avoid their undesirable build-up. These are also the reasons why prior to art Rh recovery processes known for Rh-organophosphine/bisphosphine catalytic systems are not fully applicable for Rh-organobisphosphite catalytic systems. For instance, US-A-5290743 is directed to the recovery of a hydroformylation catalyst system that contains a rhodium hydridocarbonyl tris(trisubstituted phosphine) complex, a trisubstituted phosphine and a diphosphinoalkane. In this publication no mention is made of removal of ligand degradation products.

EP-A-829300 describes a process wherein a rhodium containing aqueous solution is prepared by treating a rhodium-containing liquid from the reactor with an oxidizing agent in the presence of a recovery accelerator and an aqueous medium. In the next step, Rh is re-extracted into an organic phase by using arylphosphine ligands. A major drawback of the process as disclosed in EP-A-829300 for use with organobisphosphites is that a carboxylic acid is present as recovery accelerator in the aqueous solution, which is as hydrolysis catalyst not compatible for a re-extraction with organobisphosphites.

Several patents describe methods for the removal of organophosphite degradation products or methods for the recovery of Rh from hydroformylation process streams. However, none of them gives a complete and satisfactory procedure. For instance, US-A-5741942, US-A-5741944, US-A-5744649, US-A-5786517 and US-A-5874640 describe methods for the removal of phosphorous acid degradation products of organobisphosphites from hydroformylation process streams via aqueous extraction. However, no process is given for the simultaneous or subsequent removal of other organophosphite degradation products, such as phosphite oxides, and high-boiler byproducts. The separation of Rh from phosphite degradation products and high-boiler organic compounds present in organic reaction mixtures by using a multiple extraction process, in which at least one of the extraction steps is carried out in the presence of a lipophilic organophosphorous compound, such as mono-, bi- or multidentate lipophilic organophosphine, mono-, bi- or multidentate lipophilic organophosphite is not described or suggested in the above mentioned patents.

An organic mixture containing rhodium/non-lipophilic phosphite ligand complex, degradation products of such phosphite ligands and high-boiler organic compounds may be obtained by a hydroformylation process in which an ethylenically unsaturated compound is hydroformylated into a linear aldehyde in the presence of a catalyst system comprising rhodium and a multidentate organophosphite ligand. An example of such a hydroformylation process is described in WO-A-9733854 and WO-A-9819984 which disclosures are incorporated herein as reference. However, this invention is not intended to be limited to the recovery of rhodium complex from organic mixtures derived from these particular hydroformylation processes and it is believed that the process according to the invention can be applied to any Rh non-lipophilic or lipohilic organic phosphite based catalyst in organic mixtures derived from hydroformylation, hydrogenation, carbonylation or carboxylation processes.

An organic mixture containing rhodium/non-lipophilic phosphite ligand complex, degradation products of such phosphite ligands and high-boiler organic compounds is for example also obtained in a process as described in WO-A-9634687. This publication describes the use of membrane filtration for the separation of aldehyde hydroformylation reaction product from Rh-organobisphosphite complex catalyst and free organobisphosphite ligand and subsequent recycling the rhodium catalyst containing retentate. A disadvantage of this process is that the membrane retains all the components which have similar or higher molecular weight or size than the Rh-organobisphosphite catalyst complexes. Therefore, ligand degradation products and high-boiler organic compounds, which have similar or higher molecular weight or size than the catalyst complexes, will be recycled with the catalyst resulting in continuous accumulation of such ligand degradation products and high-boiler organic compounds in the catalyst recycle stream. This results again in a decrease in effective reactor volume and productivity.

An organic mixture containing rhodium/non-lipophilic phosphite ligand complex, degradation products of such phosphite ligands and high-boiler organic compounds is preferably treated in the process according to the invention with method 1 or method 2 which are described below.

With method 1 is meant
A) subjecting the organic reaction mixture containing Rh/non-lipophilic phosphite ligand complex, degradation products of such phosphite ligand and high-boiler organic compounds to an aqueous oxidative and/or acidic treatment, resulting in an aqueous-polar organic two-phase mixture,
B) removing the aqueous phase and optionally neutralizing the organic phase by aqueous washing or by an alternative treatment,
C) adding a solution of lipophilic phosphine and/or lipophilic phosphite in a non-polar solvent to the organic phase, which solvent is immiscible with the polar organic phase, resulting in a non-polar organic and polar organic two-phase mixture,
D) prior or after C, optionally adding a polar solvent to the polar organic phase,
E) optionally subjecting the organic two-phase mixture to a reductive treatment,
F) separating the non-polar organic phase obtained after step E,
G) after optional addition of process solvent to the non-polar organic phase obtained in F, and after optional evaporation of the non-polar solvent from the non-polar organic phase, recycling to the reactor.

With method 2 is meant
A) subjecting an organic reaction mixture containing Rh/non-lipophilic phosphite ligand complex, degradation products of such phosphite ligand and high-boiler organic compounds to an aqueous oxidative and/or acidic treatment, resulting in the formation of an aqueous-polar organic two-phase mixture,
B) removing the aqueous phase and optionally neutralizing the thus obtained organic phase by aqueous washing or by an alternative treatment,
C) adding a solution of lipophilic phosphine and/or lipophilic phosphite in a non-polar solvent to the organic phase, which solvent is immiscible with the polar organic phase, resulting in a non-polar organic and polar organic two-phase mixture,
D) prior or after C, optionally adding a polar solvent to the above obtained polar organic phase in order to facilitate phase separation.
E) prior or after C, optionally subjecting the thus obtained organic two-phase mixture to a reductive treatment,
F) separating the non-polar organic phase obtained after step E,
G) adding a non-lipohilic phosphite ligand in a polar solution which is immiscible with the non-polar organic phase obtained in step F,
H) optionally subjecting the obtained organic two-phase mixture to a reductive treatment,
I) separating the polar organic phase,
J) after optional partial evaporation of the solvents, recycling of the polar organic phase obtained in I,to the reactor.

An organic mixture containing rhodium/lipophilic phosphite ligand complex, degradation products of such lipophilic phosphite ligands and high-boiler compounds may be obtained by a hydroformylation process in which an ethylenically unsaturated compound is hydroformylated into a linear aldehyde in the presence of a catalyst system comprising rhodium and a multidentate lipophilic organophosphite ligand. An example of such a hydroformylation process is described in WO-A-9906345 which disclosure is incorporated herein as reference. With the term lipophilic ligand is meant a ligand that, in a two-phase organic solvent system formed by mixing a polar with a non-polar solvent, will be substantially distributed in the non-polar phase. An example of a lipophilic phosphite ligand is a phosphite ligand having the structure [[P(OR) (OR')O]ₙR"]ₘ wherein n is an integer from 1 to 4 and m is an integer of at least 1, wherein R, R' and R" are organic residues which may be the same or different and wherein R, R' and/or R" contain at least one C₉ to C₄₀ aliphatic group positioned as a tail extending away from the primary ligand structure rendering the ligand lipophilic.

A preferred ligand is a bidentate organic ligand [P(OR)(OR')O)₂R" containing at least one C₉ to C₄₀ aliphatic group positioned on the backbone or connecting structure of the ligand. These types of ligands, method of preparation, their specific properties and possible hydroformylation reaction embodiments are for example disclosed in WO-A-9906345 which disclosure is incorporated herein as reference.

An organic mixture containing rhodium/lipophilic phosphite ligand complex, degradation products of such phosphite ligands and high-boiler organic compounds is preferably treated in the process according to the invention with method 3 or method 4 described below.

With method 3 is meant:
A) subjecting the organic reaction mixture containing Rh/lipophilic phosphite ligand complex, degradation products of such phosphite ligand and high-boiler organic compounds to an aqueous oxidative and/or acidic treatment, resulting in an aqueous-polar organic two-phase mixture,
B) removing the aqueous phase and optionally neutralizing the organic phase by aqueous washing or by an alternative treatment,
C) optionally adding a polar solvent to the polar organic phase
D) extracting the organic phase with an immiscible non-polar organic solvent,
E) after removal of the non-polar extract adding a solution of lipophilic phosphite to the organic phase in a non-polar solvent, which is immiscible with the polar organic phase,
F) prior or after E, optionally subjecting the organic two-phase mixture to a reductive treatment,
G) separating the non-polar organic phase obtained after step F,
H) after optional addition of process solvent to the non-polar organic phase obtained in G, and after optional evaporation of the non-polar solvent from the non-polar organic phase, recycling to the reactor.

With method 4 is meant:
A) subjecting the organic reaction mixture containing Rh/lipophilic phosphite ligand complex, degradation products of such phosphite ligand and high-boiler organic compounds to an aqueous oxidative and/or acidic treatment, resulting in an aqueous-polar organic two-phase mixture,
B) removing the aqueous phase and optionally neutralizing the organic phase by aqueous washing or by an alternative treatment,
C) optionally adding a polar solvent to the polar organic phase
D) extracting the organic phase with an immiscible non-polar organic solvent,
E) after removal of the non-polar extract adding a non-lipophilic phosphite to the polar organic phase optionally in a polar solvent,
F) prior or after E, optionally subjecting the polar organic phase to a reductive treatment,
G) after optional addition of process solvent to the polar organic phase obtained in F, recycling to the reactor.

The present invention provides an improved process for the recycling of the catalyst including complete or almost complete removal of the ligand degradation products and regeneration of the catalyst. The order of the steps in the above given methods of the invention is important, although it is, for example, possible to carry out step C before step B in methods 1-2. It is also possible to leave out steps, which are indicated as optional. However, the preferred order of the steps of methods 1-4 is as given above with all the optional steps being carried out. Each of the steps of methods 1-4 can be repeated in order to increase efficiency in the Rh-recovery process. Furthermore, any of the methods 1-4 can be combined with any other in order to increase efficiency in the Rh-recovery process.

In the following the process will be explained in more detail, according to the above-described order of process steps.

Without wishing to be bound to any particular theory, it is believed that in the oxidative and/or acidic treatment (step A) of methods 1-4 of the present invention the phosphite ligand present in the rhodium/ligand complex and the optionally free phosphite ligand are destructed into so-called ligand degradation products, regardless of the fact that the phosphite is lipophilic or not in character. The oxidative treatment results in a two-phase mixture comprising an organic phase containing Rh and oxidized phosphorous compounds and an aqueous phase containing a smaller amount of hydrolyzed phosphites. The oxidative treatment can be performed with any suitable oxidant, for example oxygen or an oxygen containing gas. It is also possible to use an oxidizing substance, for example hypochlorite or a peroxide compound. This peroxide compound is an inorganic peroxide, for example hydrogen peroxide, or an organic peroxide such as t-butyl peroxide. Preferably, an inorganic peroxide or hypochlorite is used as oxidant in aqueous solution. A more preferred oxidant is hypochlorite in aqueous solution. The concentration of liguid or solid oxidant in the aqueous phase may vary between 0.01 and 60 % and depends on the nature of the oxidant and the contents of the organic phase, which can be oxidized upon the treatment. Preferably, hypochlorite is used in a concentration of between 5-15%. Similarly, the volume of aqueous phase used in the oxidative treatment may vary in all practical ranges, for instance, between 5 and 2000 volume % of the organic phase. Preferably, the volume of aqueous phase is equal to or less than the organic phase used in the oxidative treatment. The ligand destruction can also be carried out by performing an acidic treatment. An acidic treatment will result in a two-phase mixture comprising an organic phase containing Rh and an aqueous phase containing acidic phosphorous compounds. In case of lipophilic ligand systems (methods 3 and 4), the organic phase will contain a substantial amount of acidic and non-acidic lipophilic degradation products. The acidic treatment can be performed using inorganic acids such as for example sulfuric, phosphoric or nitric acid or using organic acids such as for example sulfonic acid. Preferred are acids of which the corresponding bases do not coordinate strongly to the rhodium atom, for example sulfuric, phosphoric or nitric acid. More preferred is sulfuric acid. The concentration of acid in the aqueous phase may vary between 0.0001 and 80 % and it depends on the nature of the acid and the contents of the organic phase, which can be hydrolyzed upon the acidic treatment. Preferably, sulfuric acid is used between 5 and 15% concentration. Similarly, the volume of aqueous phase used in the acidic treatment may vary in all practical ranges, for instance, between 5 and 2000 volume % of the organic phase. Preferably, the volume of aqueous phase is equal to or less than the organic phase used in the acidic treatment.

The oxidation and/or acidic treatment in methods 1-4 is performed under conditions effective to completely or almost completely destruct the amount of non-lipophilic or lipophilic phosphite ligand, present as free and/or complexed ligand including its partially degraded forms in the mixture to be treated in the present process. Preferably, the oxidation and/or acidic treatment is performed such that the amount of non-destructed phosphite ligand in the mixture of destructed ligand and rhodium is less than about 5% of the initial phosphite ligand content before the treatment. The amount of non-destructed ligand can be measured by any technique known to a man skilled in the art, for example ³¹P nuclear magnetic resonance spectroscopy (³¹P NMR) of inductive coupled plasma mass spectroscopy (ICP-MS).

The oxidative and/or acidic treatment of methods 1-4 can be performed over a wide temperature range. A preferred temperature range for this step is from about 20 °C to about 120 °C; a more preferred range is from 40 °C to about 120 °C. More specific, the more preferred temperature to perform an oxidative treatment is ambient temperature and the more preferred temperature to perform an acidic treatment is between 80 °C and 110 °C.

The necessary pressure to perform the oxidative and/or acidic treatment can be determined by anyone skilled in the art and depends on the used reactants. The preferred pressure applied in the oxidative and/or acidic treatment is atmospheric pressure.

The residence time in the oxidative and/or acidic treatment can be varied between 1 minute and 5 hours and depends essentially on the applied temperature. Preferably, the residence time is between 30 minutes and 2 hours.

Subsequently to the oxidative and/or acidic treatment, the aqueous phase is removed as waste from the organic phase subsequent to the oxidative or acidic treatment. The obtained organic phase can be washed with an aqueous solution or portions of an aqueous solution. As mentioned above the aqueous washing is optional in methods 1-4. Whether the aqueous washing is required depends on the nature and amount of reagents used in the oxidative and/or acidic treatment. The primary goal of aqueous washing is to remove harmful residual oxidants or acidic compounds, if these are present, from the organic phase. However, this neutralization step, when necessary, can be achieved by other means than aqueous washing,for example, by contacting the organic phase with an appropriate soluble or insoluble solid scavenger. Similarly, soluble or insoluble liguids or gases can also be used for this purpose. For instance, when an acidic treatment is used it is believed that a neutralizing washing step using a basic aqueous solution increases the efficiency of the Rh-recovery process. The use of an insoluble solid or liquid base for this purpose gives similarly good results. Therefore, the neutralization of the organic phase after the acidic treatment can be carried out by using an aqueous or another heterogeneous base. The neutralization can also be carried out by using a solid, a liquid or a gaseous base, which is soluble in the organic phase. Therefore, the nature of the base used for neutralization is not crucial and it can be selected by anyone skilled in the art. For example, inorganic bases such as soda, sodium bicarbonate,ammonia etc. can be used either in heterogeneous forms or aqueous solutions. Water-soluble amines or other organic bases can also be used either in aqueous solutions or in other heterogeneous forms including ion exchange resins. Preferably, an aqueous or another heterogeneous base is used in method 1 and 4 when necessary. In method 2-3 no preference is made for the base. The neutralization of the organic phase after the oxidative treatment can be carried out by using water and an aqueous solution or another heterogenous form of a reductant. The neutralization can also be carried out by using a solid, a liquid or a gaseous reductant, which is soluble in the organic phase. The nature of reductant is not crucial and it can be selected by anyone skilled in the art. For example, copper(I) and iron(II)-salts can be used can be used either in heterogeneous forms or aqueous solutions. Hydroquinone can also be used as organic soluble reductant. Preferably, an aqueous or another heterogeneous reductant is used in method 1 and 4, when necessary. In methods 2-3, no preference is made for the reductant.

Subsequently to the removal of the aqueous phase or the neutralization of the organic phase, a lipophilic phosphine and/or phosphite ligand is added to the organic phase in methods 1-3. Lipophilic phosphite ligand which can be used are represented by the structure [[P(OR)(OR')O]ₙR"]ₘ wherein n is an integer from 1 to 4 and m is an integer of at least 1, wherein R, R' and R" are organic residues which may be the same or different and wherein R, R' and/or R" contain at least one C₉ to C₄₀ aliphatic group positioned as a tail extending away from the primary ligand structure rendering the ligand lipophilic.

A preferred ligand is a bidentate organic ligand [P(OR)(CR')O]₂R" containing at least one C₉ to C₄₀ aliphatic group positioned on the backbone or connecting structure of the ligand. These types of ligands, method of preparation, their specific properties and possible hydroformylation reaction embodiments are for example disclosed in WO-A-9906345 which disclosure is incorporated herein as reference. More preferably, in step C of method 1, the added lipophilic bisphosphite ligand differs only in the length of aliphatic group positioned at the backbone or connecting structure from the non-lipophilic bisphosphite ligand, which is present in the organic mixture before the treatment. More preferably, in step E of method 3, the added lipophilic bisphosphite ligand is the same as the lipophilic bisphosphite ligand, which is present in the organic mixture before the treatment. Also in step C of method 2 a lipophilic ligand is added to the organic phase. The lipophilic ligand which can be used in this step can be a phosphine or phosphite derivative represented by the structure [(PRR')ₙR"]ₘ or [[P(OR) (OR')O]ₙR"]ₘ, respectively, wherein n is an integer from 1 to 4 and m is an integer of at least 1, wherein R, R' and R" are organic residues which may be the same or different and wherein R, R' and/or R" contain at least one C₉ to C₄₀ aliphatic group positioned as a tail extending away from the primary ligand structure rendering the ligand lipophilic. A preferred ligand for use in step C of method 2 is a lipophilic monodentate ligand represented by the structure P(RR'R") or P[(OR)(OR')(OR")], respectively, wherein R, R' and R" are organic residues which may be the same or different and wherein R, R' and/or R" contain at least one C₉ to C₄₀ aliphatic group positioned as a tail extending away from the primary ligand structure. The lipophilic ligand is preferably added in an amount effective to recover rhodium as rhodium/lipophilic ligand complex. In order to regenerate rhodium as rhodium/ligand complex, the amount of ligand to be added in step C of method 1 and in step E of method 3 is at least 1 mol ligand per mol rhodium. Preferably, 1-10 mol ligand per mol rhodium is added. More preferably, 1-4 mol ligand per mol rhodium is added. The amount of lipophilic ligand added in step C of method 2 is at least 2 mol ligand per mol rhodium. Preferably, 2-50 mmol ligand per mol Rh is added in this step. More preferably, 2-15 mmol ligand per Rh is added in step C of method 2. The lipophilic ligands can be added directly to the obtained organic phase of steps A-B of methods 1-3. Preferably, the lipophilic ligand is added dissolved in an extraction solvent, which is immiscible with the obtained organic phase of steps A-B of methods 1-3. Preferred extraction solvents for this purpose are alkanes for example hexane, isooctane, cyclohexane and the like. Most preferred is the use of apolar solvents such as hexane. The amount of extraction solvent will depend on the partition coefficient of for example rhodium which is dependent on the extraction solvent. The amount of extraction solvent should be sufficiently high in order that two separate liquid phases exist during extraction. Therefore, the extraction solvent can be used in all practical ranges, for instance, between 5 and 2000 volume % of the polar organic phase of steps A-B of methods 1-3. Preferably, the volume of the extraction solvent is similar to the organic phase obtained in steps A-B of methods 1-3.

In order to facilitate phase separation in step F of methods 1-2, in steps D and G of method 3 and in step D of method 4, optionally a polar organic solvent can be added to the organic phase obtained in steps A-B of methods 1-4. The addition of this polar co-solvent can be carried out prior or after the addition of the lipophilic phosphite or phosphine solution. Preferred polar co-solvents for this purpose are highly polar solvents such as for example acetonitrile and dimethylsulfoxide. More preferred is the addition of acetonitrile. The amount of polar co-solvent optionally used can vary in any practical ranges. Preferably, when the polar co-solvent is used its volume is not more than the organic phase obtained in steps A-B of methods 1-4.

In step E of methods 1-2, step F of method 3-4 and step H of method 2, the obtained organic two-phase mixture is optionally subjected to a reductive treatment. As indicated, it is also possible to carry out the reductive treatment, when necessary, before the addition of the non-polar extraction solvent and ligand. Preferably, the reductive treatment is carried out after the addition of ligand. Generally, it is preferable to carry out the reductive treatment in step E of methods 1-2, step F of method 3-4 and in step H of method 2. Without wishing to be bound to any particular theory, it is believed that the use of reductive treatment in methods 1-4 increases the efficiency of the Rh-recovery process. Without wishing to limit to theory, it is thought that in step A of methods 1-4 the valency of rhodium valency is increased, hereby decreasing the co-ordinating properties of Rh. Upon the reductive treatment the valency of Rh is decreased, hereby increasing the coordination properties of Rh. Due to these effects, it is possible to separate the destructed ligand compounds from Rh and to regenerate the active Rh-complex.

The reductive treatment in step E of methods 1-2, step F of methods 3-4 and step H of method 2 can be performed with several agents for example hydrogen, an inorganic compound for example sodium borohydride, an organic compound as for example formaldehyde, also a hydrogen containing gas can be used as for example syngas (gas containing carbon monoxide and hydrogen). Preferred is hydrogen or hydrogen containing gas. In particular syngas is preferred. Preferably, the reductive treatment is performed at a pressure of between 0.1 - 10 MPa and a temperature of 20 °C - 150 °C. More preferred is a temperature of 60 °C - 100 °C and a pressure of 1 - 5 MPa. In case syngas is used, the molar ratio CO and H₂ is preferably between 10:1 and 1:10 and more preferably about 1:1.

The residence time in step in the reductive treatment can be varied between 5 minutes and 5 hours and depends essentially on the applied pressure and temperature. Preferably, the residence time is between 10 minutes and 1 hour.

A non-limiting example of possible reaction conditions for the reductive treatment is as follows: heating up the rhodium containing solution obtained from the extraction step for 1 hour under 3 MPa of CO/H₂ at 100 °C.

After the reductive treatment in step E of methods 1-2, step F of method 3 and step H of method 2 a polar phase is separated from a non-polar phase in step F of methods 1-2, step G of method 3 and step I of method 2. The temperature applied during these phase separation steps is generally between 0 °C and 80 °C. The pressure is generally between 0.1 and 0.5 MPa. Preferably, step F of methods 1-2, step G of method 3 and step I of method 2 are carried out between 20 °C and 40 °C under atmospheric pressure.

After the phase separation, the phase, which is rich in Rh is further processed in step G of methods 1-2,step H of method 3 and step J of method 2. As said above, each step of methods 1-3 can be repeated in order to increase efficiency in the Rh-recovery process. For example, when step C of methods 1-2 or step E of method 3 (addition of a new portion of liphophilic ligand in an extraction solvent) is repeated after phase separation in step F of method 1-2 or step I of method 3,respectively, the new extraction can be carried out with or without repeating the reductive treatment. Preferably, the reductive treatment is repeated, when step C of methods 1-2 or step E of method 3 are repeated.

In step D of methods 3-4 the organic phase, which is obtained after acidic or oxidative treatment, is extracted by using an extraction solvent. The purpose of the extraction is to remove lipophilic phosphite degradation products from the organic phase to be treated in step D of methods 3-4. As for extraction solvent any apolar solvent can be used which is immiscible with the organic phase to be extracted. (As said above the phase separation can be facilitated by adding a polar co-solvent to the organic phase prior to extraction, step C of methods 3-4). Preferred extraction solvents for this purpose are alkanes or mixtures of alkanes with arenes,for example, hexane, mixtures of hexane with toluene, isooctane, cyclohexane and the like. Most preferred is the use of pure hexane or its concentrated solutions. It is also preferable to carry out step D and E of method 3 by using the same immiscible solvent or solvent composition. The amount of extraction solvent used will depend on the partition coefficient of lipophilic phosphite degradation products, which is dependent on the extraction solvent. The amount of extraction solvent should be sufficiently high in order that two separate liquid phases exist during extraction. Therefore, the extraction solvent can be used in all practical ranges, for instance, between 5 and 2000 volume % of the polar organic phase of steps A,B or C of methods 3-4. Preferably, the volume of the extraction solvent is similar to the organic phase obtained in steps A,B or C of methods 3-4. The extraction can be carried out with or without stirring. Preferably, the obtained two-phase system is stirred at least for 5 minutes before phase separation. The temperature applied during the extraction in step D of methods 3-4 is generally between 0 °C and 80 °C. The pressure is generally between 0.1 and 0.5 MPa. Preferably, step D of methods 3-4 is carried out between 20 °C and 40 °C under atmospheric pressure.

In step G of method 2 a non-lipophilic organophosphite ligand and an immiscible polar solvent is added to the non-polar organic phase obtained in the previous step. Preferably, the non-lipophilic organophosphite ligand is added dissolved in a polar solvent, which is immiscible with the apolar phase. In step E of method 4 a non-lipophilic organophosphite ligand is added optionally in a polar solvent. In step G of method 2 and step E of method 4, the non-lipophilic ligand is preferably added in an amount effective to regenerate rhodium as rhodium/non-lipophilic ligand complex. In order to regenerate rhodium as rhodium/ligand complex, the amount of ligand to be added in step G of method 2 and in step E of method 4 is at least 1 mol ligand per mol rhodium. Preferably, 1-10 mol ligand per mol rhodium is added. The non-lipophilic phosphite ligand which can be used are represented by the structure [[[P(OR) (OR')O]ₙR"]ₘ wherein n is an integer from 1 to 4 and m is an integer from at least 1, wherein R, R' and R" are organic residues.

A preferred ligand is a bidentate organic ligand P(OR)(OR')O]₂R". More preferably, in step G of method 2, the added non-lipophilic bisphosphite ligand is the same as the non-lipophilic bisphosphite ligand, which is present in the organic mixture before the treatment. In step E of method 4, the added non-lipophilic bisphosphite ligand differs preferably only in the length of aliphatic group positioned at the backbone or connecting structure from the lipophilic bisphosphite ligand, which is present in the organic mixture before the treatment.

Beside the non-lipophilic ligand, a solvent is added in step G of method 2. As for added solvent any polar solvent can be used which is immiscible with the non-polar phase obtained in the previous step (step F of method 2). Preferred solvents for this purpose are polar solvents such as esters,aldehydes or nitriles. More preferred is the use of aldehyde-esters such as 5-formylmethylvalerate or acetonitrile. The amount of the polar solvent used will depend on the partition coefficient of Rh-complex, which is dependent on the extraction solvent. The amount of the polar solvent should be sufficiently high in order that two separate liquid phases exist during extraction. Therefore, the extraction solvent can be used in all practical ranges, for instance, between 5 and 2000 volume % of the polar organic phase of step F of method 2. Preferably, the volume of the extraction solvent is similar to the organic phase obtained in step F of method 2.

The non-lipophilic ligand can be added alone or in a solvent in step E of method 4. As for solvent any solvent can be used which is miscible with the polar phase obtained in the previous step (step D of method 4). Preferred solvents for this purpose are solvents such as toluene, esters, aldehydes or nitriles. More preferred is the use of aldehyde-esters such as 5-formylmethylvalerate or toluene.

In step G of method 1, step J of method 2, step H of method 3 and step G of method 4, the regenerated Rh-ligand complex is recycled to the reactor.

The organic mixture which is treated in the process of the invention is generally removed continuously or intermittently from a hydrogenation, hydroformylation or carbonylation process utilizing Rh-bi- or multidentate phosphite based catalyst. The mixture is removed from some point in the catalyst recycle stream. Although, it is not the primary target of the present invention, it believed that the concept of the invention is also applicable to Rh/monodentate phosphite systems.

The concentration of rhodium complex in the mixture to be treated is not critical. The concentration will generally be between 100 and 5000 ppm rhodium.

The concentration of ligand degradation products in the mixture to be treated may vary from 0.01 - 10 wt.% and can possibly contain high-boiler compounds such as for example aldol condensation products. The molar ratio of P- and Rh-content in the mixture to be treated may vary between 0.01:1 to 1000:1. Preferably, the P to Rh molar ratio in the mixture to be treated is more than 20:1. When the molar ratio of P to Rh is lower than 20:1 in the mixture to be treated, it is preferable to add a phosphorus compound to the mixture to bring the P to Rh molar ratio above 20:1. For this purpose, for example triphenylphosphine or tris(orthotolyl)phosphine can be added to the mixture before the treatment. Preferably, tris(orthotolyl)phosphine is added, when the molar ratio of P to Rh is lower than 20:1 in the mixture to be treated. It has been found that the presence of a organophosphine compound in the organic mixture to be treated enhances the extraction efficiency in the process of the invention.

The invention will further be elucidated by means of the following, non-limiting examples. In these examples several different organic solutions were used for testing the efficiency of Rh-recovery.

Test solution A was obtained from a hydroformylation reactor after 190 hours of continuous hydroformylation of an approximately 10:1 mixture of methyl-3-pentenoate and methyl-2-pentenoate catalyzed by a Rh-naphthol-3 based catalyst. Naphthol-3 has the following structure:

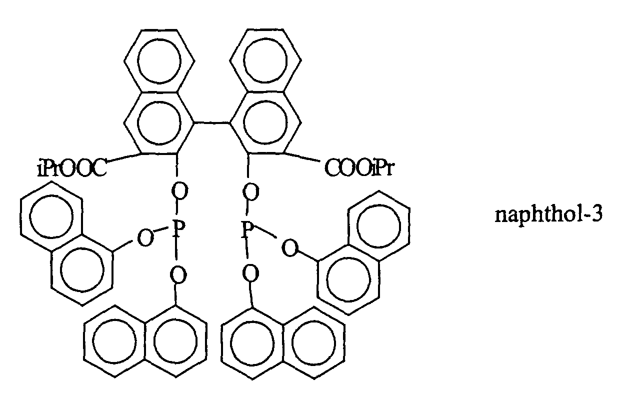

The hydroformylation was carried out at 95 °C under 5 bar a gas mixture of CO/H₂= 1.1/1 by continuous feeding of the mixture of methylpentenoates and a ligand make-up solution in toluene containing naphthol-3 and tris(orthotolyl)phosphine. The formed C6-aldehyde products were continuously removed via vacuum distillation and the catalyst was recycled from the bottom of the distillation column to the reactor maintaining a constant, approximately 240 ppm Rh level in the reactor. After 190 hours of such hydroformylation, the contents of the reactor were removed. Subsequently, most of the toluene (solvent) and methylpentenoates and approximately the half of C6-aldehydes were removed from this solution by carrying out vacuum distillation at 0.5 kPa and 105 °C. The obtained yellow solution, which contained 672 ppm of Rh and 3780 ppm of P as determined by ICP, was used for Rh-recovery tests below as test solution A. Test solution A contained 5-formylmethylvalerate as major component beside isomeric aldehydes and 13.7 % of high-boiler compounds including aldol-products of these aldehydes as determined by gas chromatography. As for P-content, ³¹P NMR and HPLC indicated the presence of free and coordinated naphthol-3 ligand, as well as its degradation products such as naphthol-3 mono- and dioxide and a monodentate phosphite degradation product The ratio of degraded naphthol-3 ligand to non-degraded naphthol-3 ligand wass 3:1. Beside some other minor monophosphite derivatives, the solution contained a substantial amount of tris(orthotolyl)phosphine and its oxide (in a concentration of about 2.6 wt.%).

Test solution B was obtained in a manner similar to that described above for test solution A, except that pure methyl-3-pentenoate was used instead of a mixture of methylpentenoates and the continuous hydroformylation was carried out for 256 hours. In this case only toluene and the unreacted methyl-3-pentenoate were removed from the reactor contents after the hydroformylation by carrying out vacuum distillation at 0.8 kPA at 100 °C. The obtained yellow solution, which contained 234 ppm of Rh and 1070 ppm of P as determined by ICP, was used for Rh-recovery tests below as test solution B. Test solution B contained 5-formylmethylvalerate as major component beside isomeric aldehydes and 5.0 % high-boiler compounds including aldol-products of these aldehydes as determined by gas chromatography. As for P-content, ³¹P NMR and HPLC showed similar composition to that given above for solution A.

Test solution C was obtained by concentrating a portion of test solution B by vacuum distillation at 105 °C under 0.5 kPa. The obtained concentrated solution, which contained 682 ppm of Rh and 3120 ppm of P, was used as test solution C for Rh-recovery experiments. Test solution C contained about 14.5 % high-boiler compounds.

Test solution D was prepared by the following method: 54 mg of Rh(CO)₂(Acac) (Acac = acetylacetonate) and 525 mg Fat-BINAP-2 was suspended in 24 ml of pure 5-formylmethylvalerate under N₂. Fat-BINAP-2 has the following structure:

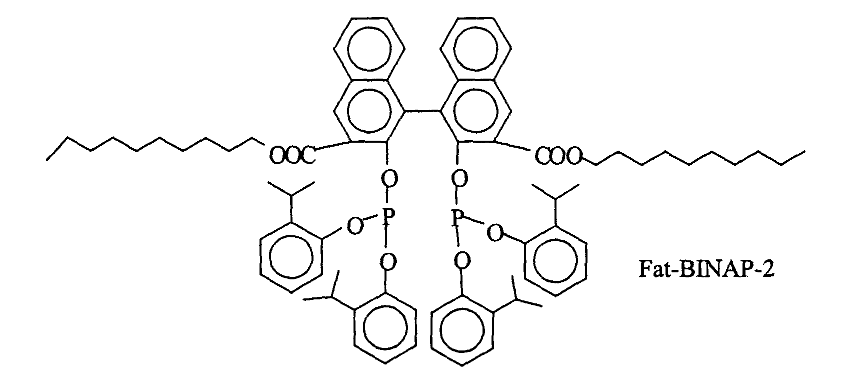

An additional 10 ml amount of 5-formylmethylvalerate was then added containing 10 w% of aldol-product of 5-formylmethylvalerate. In order to facilitate the dissolution of the lipophilic bisphosphite, 8 ml of methyl-3-pentenoate was added to the mixture, upon which a clear yellow solution was formed. Then the solution was autoclaved for one hour at 100 °C under 30 bar of CO/H₂= 1/1. After cooling and releasing pressure under N₂, the obtained solution was used for Rh-recovery test below as test solution C. Test solution C contained 494 ppm of Rh and 595 ppm of P as determined by ICP. As for P-content, ³¹P NMR indicated that close to one half of the added fat-BINAP was present coordinated to Rh in HRh(CO)₂(fat-BINAP-2)complex. The rest of the P-content consisted of uncoordinated fat-BINAP-2 and small amounts of its mono and dioxide derivatives.

### Example 1

A 30 ml amount (31.8g) of test solution A was heated with 40 ml of an aqueous solution containing 5 w% of sulfuric acid with stirring at 100 °C for 1 hour under air by using a reflux condenser. Before, during and after the heating a two-phase mixture was present. After the heating, about 20 ml of organic phase could be separated from the aqueous-phase, which went on the top during separation. A ³¹P NMR spectrum taken from the organic phase showed no coordinated or free bis- or monophosphite content present. The obtained organic phase was washed with 2x30 ml portions of saturated aqueous sodiumbicarbonate solution until gas development ceased. The aqueous washings were united with the first aqueous phase. ICP analysis from the united aqueous phases showed less than 5 ppm Rh-content, which corresponded to less than 1.7 % of total Rh-loss into the aqueous phases. The organic phase was then degassed by using 6 cycles of vacuum and readmission of N₂. Next, 30 ml of degassed acetonitrile was added to the organic phase followed by 583 mg of fat-BINAP-2 (approximately 3 mmol bisphosphite per mol Rh) and 90 ml of hexane. The obtained mixture was autoclaved for 1 hour at 100 °C under 30 bar CO/H₂ =1/1. After cooling back and venting under N₂, the obtained two-phase mixture was removed from the autoclave and the almost colorless hexane phase at the top was separated at room temperature. Then, the residual polar phase, which was about 40 ml in volume, was washed with 30 ml of degassed hexane at room temperature. The hexane washing was united with the first hexane extract. ICP analysis showed less than 15 ppm Rh left in the residual polar organic phase, which corresponded to a maximum of 2 % Rh-loss into the waste polar organic phase. Thus the total efficiency of the Rh-extraction from test solution A to hexane was higher than or equal to 96 %, which was confirmed by taking sample from the united hexane phase for ICP Rh-analysis. ³¹P NMR spectrum taken from the hexane solution showed only the presence of HRh(CO)₂(Fat-BINAP-2) as Rh complex, which is thought to be a catalytically active species. About 2/3^{rd} of the added Fat-BINAP-2 was present partly as unchanged and partly as degraded to monophosphite (hydrolyzed and oxidized). The hexane extract showed also the presence of a significant amount of tris(orthotolyl)phosphine and its oxide, which were obviously not destructed upon the acidic treatment and thus these were partly redistributed into the hexane extract. However, the concentration of the phosphine derivatives was at least 4-times higher in the residual polar phase than in the hexane phase. GC analysis showed a distribution of approximately 85% and 15% of high-boiler compounds in the residual polar and the obtained united hexane phase, respectively. The amount of high-boiler compounds removed with the aqueous phase was not determined. Example 1 demonstrates that method 1 using acidic treatment is an efficient process for the separation of phosphite degradation products and high-boiler compounds from Rh.

### Example 2

A 30 ml amount (30.6 g) of test solution C was treated with 40 ml of an aqueous solution containing 6.5 w% of sodium hypochlorite with gentle stirring at room temperature for 2 hours under air. During and after the treatment a two-phase mixture was present. In order to facilitate phase separation 30 ml of toluene was added to the mixture after the treatment. About 60 g of organic phase was separated then, which went on the top during separation. A ³¹P NMR spectrum taken from the organic phase showed only a small amount of tris(orthotolyl)phosphine present, which was not destructed. All the phosphite components were destructed mostly to oxide derivatives. The obtained organic phase was washed with 2x 20 ml portions of aqueous brine solution. The aqueous washings were united with the first aqueous phase. ICP analysis from the united aqueous phases showed less than 5 ppm Rh- content, which corresponded to less than 1.9 % of total Rh-loss into the aqueous phases. The organic phase was then degassed by using 6 cycles of vacuum and readmission of N₂. Next, 25 ml of degassed acetonitrile was added to the organic phase followed by 390 mg of fat-BINAP-2 (approximately 2 mmol bisphosphite per mol Rh) and 35 ml of hexane. The obtained two-phase mixture was stirred for 5 minutes under N₂. Approximately 28 ml hexane phase was then separated on the top. ICP analysis from this phase showed 160 ppm Rh, which corresponded to 19.3 % Rh extraction. To the separated polar organic phase (~90 ml) were added again new portions of 390 mg of fat-BINAP-2 and 70 ml of hexane. The mixture obtained two-phase mixture was autoclaved for 1 hour at 100 °C under 30 bar CO/H₂ =1/1. After cooling back and venting under N₂, the obtained two-phase mixture was removed from the autoclave and the almost colorless hexane phase (64 ml) at the top was separated at room temperature. ICP analysis from the hexane phase showed 270 ppm Rh, while in the residual polar organic phase only 32 ppm Rh was left. This corresponded to 71.6 % Rh extraction efficiency in the second extraction step. In total, 91.0 % of the Rh was extracted from the polar organic phase by the above described hexane extractions and 7.1% of Rh left in the residual polar phase. ³¹P NMR spectrum taken from the second hexane extract showed only the presence of HRh(CO)₂(Fat-BINAP-2) as Rh complex, which is thought to be a catalytically active species. About 60 % of Fat-BINAP-2 was present as unchanged, free ligand and only 6% of fat-BINAP-2 was degraded to monophosphite. The first and second hexane extracts showed also the presence of a some tris(orthotolyl)phosphine and its oxide by ³¹P NMR. However, the concentration of the latter derivatives was at least 4-times higher in the residual polar phase than in the first and second hexane extract, as judged by ³¹P NMR. The residual polar phase showed the dominating presence of oxide derivatives of tris(orthotolyl)phosphine and various phosphites. The hexane extracts were united then for GC analysis, which showed a distribution of approximately 87% and 13% of high-boiler compounds in the residual polar and the obtained united hexane phase, respectively. The amount of high-boiler compounds removed with the aqueous phase was not determined. Example 2 demonstrates that method 1 by using an oxidative treatment is an efficient process for the separation of phosphite degradation products and high-boiler compounds from Rh, as well as for the regeneration of the Rh-bisphosphite based catalyst. Furthermore, Example 2 also demonstrates the efficiency of the reductive treatment in the extraction process.

### Example 3

A 30 ml amount (30.2 g) of test solution B was treated with 30 ml of an aqueous solution containing 6.5 w% of sodium hypochlorite with gentle stirring at room temperature for 2 hours under air. During and after the treatment a two-phase mixture was present. In order to facilitate phase separation 6 ml of toluene was added to the mixture after the treatment. About 36 g of organic phase was separated then, which went on the top during separation. A ³¹P NMR spectrum taken from the organic phase showed that all the phosphine and phosphite components were destructed mostly to oxide derivatives. The obtained organic phase was washed with 30 ml of water. The aqueous washing was united with the first aqueous phase. ICP analysis from the united aqueous phase showed less than 5 ppm of Rh-content, which corresponded to less than 4.0 % of total Rh-loss into the aqueous phases. The organic phase was then degassed by using 6 cycles of vacuum and readmission of N₂. Next, 45 ml of degassed hexane was added to the organic phase followed by 600 mg of greasy phoshine P(C₆H₄-*p*-OC₁₂H₂₅)₃ (approximately 10 mmol P per mol Rh). The obtained two-phase mixture was stirred for 5 minutes under N₂. Approximately 52 ml yellow hexane phase was then separated on the top. ICP analysis from this phase showed 15 ppm Rh, which corresponded to 7 % Rh extraction. ³¹P NMR spectrum taken from the hexane phase showed mostly the presence of uncoordinated greasy phosphine with a small amount of tris(orthotolyl)phosphine oxide. ³¹P NMR from the polar phase showed no greasy phosphine derivatives present but a large amount of oxide derivatives of various phosphites and of tris(orthotolyl)phosphine. Then the separated hexane and polar organic phases were mixed again and after the addition of 100 mg of fresh greasy phosphine the mixture was autoclaved for 1 hour at 100 °C under 30 bar CO/H₂ =1/1. After cooling back and venting under N₂, the obtained two-phase mixture was removed from the autoclave and the brown-red hexane phase (50 ml) at the top was separated at room temperature. ICP analysis from the hexane phase showed 151 ppm Rh, while in the polar organic phase (20 ml) only 28 ppm Rh was left. This corresponded to 91 % Rh extraction efficiency in the extraction step in comparison with the obtained 7% without utilizing a reductive treatment. ³¹P NMR spectrum taken from the hexane extract, showed mostly the presence of greasy phosphine oxide with a little coordinated and free greasy phosphine left. ³¹P NMR spectrum taken from the organic phase showed no significant changes compared to the analogous spectrum recorded before the reductive treatment. Then, to the separated polar organic phase (16 ml) were added again 30 ml of degassed hexane and 400 mg of fresh greasy phosphine. The mixture was autoclaved for 1 hour at 100 °C under 30 bar CO/H₂ =1/1. After cooling back and venting under N₂, the obtained two-phase mixture was removed from the autoclave and the brown-red hexane phase (32 ml) at the top was separated at room temperature. ICP analysis from the hexane phase showed 10 ppm Rh, while in the polar organic phase (14 ml) 21 ppm Rh was left. This corresponded to a total of 96 % of Rh extraction efficiency including all extraction steps. ³¹P NMR spectrum taken from the second hexane extract, showed mostly the presence of free greasy phosphine. ³¹P NMR spectrum taken from the organic phase showed no significant changes compared to the analogous spectrum recorded before the reductive treatment. Next, 30 ml of a mixture of 5-formylmethylvalerate and its isomers were added to the first hexane extract (44ml) under N₂, upon which a two-phase mixture was obtained with about 46 ml of aldehyde phase and 28 ml of hexane phase. An amount of 219 mg of BINAP-2 was added then and the mixture was stirred for 5 minutes under N₂. BINAP-2 has the following structure:

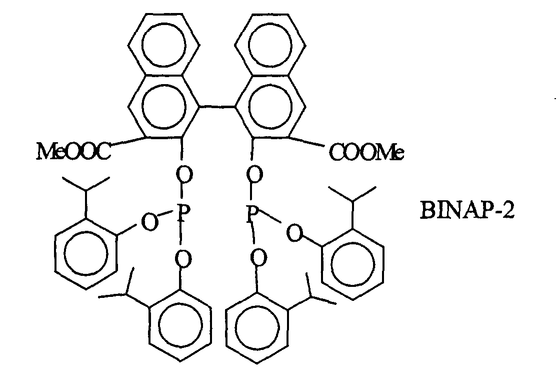

A sample taken from the aldehyde phase for ICP showed only a marginal Rh-content. The two-phase mixture was then autoclaved for 1 hour at 100 °C under 30 bar of CO/H₂ =1/1. After cooling back and venting under N₂, the obtained two-phase mixture was removed from the autoclave and the yellow aldehyde phase (50 ml) at the bottom was separated from the hexane phase (22 ml) at room temperature. ICP analysis from the polar organic phase showed 35 ppm Rh, while in the hexane phase 213 ppm Rh left. This corresponded to 38 % efficiency of the extraction from the hexane phase to the aldehyde phase. ³¹P NMR spectrum taken from the aldehyde phase showed mostly the presence of uncoordinated BINAP-2 without any of its degradation products. Beside BINAP-2, some greasy phosphine oxide was also detected. However, the concentration of the latter derivative was much less that that measured in the hexane phase. Example 3 demonstrates that method 1 and 2 by using an oxidative treatment is an efficient process for the separation of phosphite degradation products from Rh, as well as for the regeneration of the Rh-bisphosphite based catalyst. Furthermore, Example 3 also demonstrates the efficiency of the reductive treatment in the extraction process.

### Example 4

A 31 ml amount (33.21 g) of test solution A was treated with 30 ml of an aqueous solution containing 6.5 w% of sodium hypochlorite with gentle stirring at room temperature for 2 hours under air. During and after the treatment a two-phase mixture was present. In order to facilitate the complete oxidation of phosphine and phosphite components an additional amount of 15 ml of 13 % sodium hypochlorite solution was added and the mixture was stirred for an additional half an hour. A ³¹P NMR spectrum taken from the organic phase showed only the presence of oxides including those of tris(orthotolyl)phosphine and various phosphites. After removing the aqueous bleach solution, which went on the top during separation, the organic phase was washed with 2x25 ml of diluted (2-3%) aqueous sodiumbicarbonate solution. After the aqueous washings 28 ml of organic phase was obtained. The organic phase was then degassed by using 6 cycles of vacuum and readmission of N₂. Next, 20 ml of degassed acetonitrile was added to the organic phase followed by 35 ml of a solution of 367 mg of greasy phoshine P(C₆H₄-*p*-OC₁₂H₂₅)₃ (approximately 2 mmol P per mol Rh) in a mixture of hexane/toluene = 4/1. The obtained two-phase mixture was stirred for 5 minutes under N₂. Approximately 28 ml of yellow hexane phase was then separated from the top. A new portion of 30 ml solution of 367 mg of greasy phoshine P(C₆H₄-*p*-OC₁₂H₂₅)₃ in a mixture of hexane/toluene = 4/1 was added to the polar phase(53 ml). The two-phase mixture was autoclaved for 1 hour at 100 °C under 30 bar CO/H₂ =1/1. After cooling back and venting under N₂, the obtained two-phase mixture was removed from the autoclave and the brown-red hexane phase (29 ml) at the top was separated at room temperature and united with the first hexane extract. The reductive extraction was then repeated at 100 ° C under 30 bar of CO/H₂ =1/1 after adding a new portion of 30 ml solution of 367 mg of greasy phoshine P(C₆H₄-*p*-OC₁₂H₂₅)₃ in a mixture of hexane/toluene = 4/1 to the polar phase(54 ml) After cooling back and venting under N₂, the obtained two-phase mixture was removed from the autoclave and the brown-red hexane phase (30 ml) at the top was separated at room temperature and united with the previous hexane extracts. In this manner, 86 ml of united hexane extract was obtained, which contained 261 ppm of Rh. The residual organic phase (53 ml) contained 63 ppm of Rh, which corresponds to a total of 85 % efficiency in the previous Rh-extraction steps. ³¹P NMR spectrum taken from the hexane extract, showed mostly the presence of greasy phosphine oxide with some coordinated and free greasy phosphine. (The oxide content was partly due to an initial 33 % oxide present in the starting material of greasy phosphine). Beside the greasy phosphine oxides small amounts other oxides such as those of tris(orthotolyl)phosphine and various phosphites were also detected. However, the concentration of the latter derivatives was about 10 times higher in the residual polar phase than in the united hexane phase. Next, 30 ml of a mixture of 5-formylmethylvalerate and its isomers were added to the united hexane extract (84ml) together with 300 mg of BINAP-2 under N₂. The two-phase mixture was then autoclaved for 1 hour at 100 °C under 30 bar of CO/H₂ =1/1. After cooling back and venting under N₂, the obtained two-phase mixture was removed from the autoclave and the yellow aldehyde phase (41 ml) at the bottom was separated from the hexane phase (66 ml) at room temperature. ICP analysis, showed 96 ppm Rh in the aldehyde phase, which corresponds to 27.5 % Rh-extraction efficiency for the latter extraction step. ³¹P NMR spectrum taken from the aldehyde phase showed mostly the presence of uncoordinated BINAP-2 as for BINAP-2 content. Beside coordinated BINAP-2, about 10 % of the BINAP-2 content was present in HRh(CO)₂(BINAP-2)complex, which is thought to be a catalytically active species. Beside, the above mentioned BINAP-2 derivatives some greasy phosphine oxide and smaller amounts various phosphite oxides were also detected. Next, the separated red-brown hexane extract was recycled into the autoclave with 30 ml of a new portion of 5-formylmethylvalerate and its isomers together with 300 mg of a new portion of BINAP-2. The mixture was autoclaved again for half an hour at 100 °C under 30 bar of CO/H₂ =1/1. After cooling back and venting under N₂, the obtained two-phase mixture was removed from the autoclave and the reddish hexane phase (36 ml) at the top was separated at room temperature from the yellow aldehyde phase (60 ml). The second aldehyde extract was united with the first one. In order to compensate the hexane loss into the aldehyde phase, a new portion of 30 ml of a mixture of hexane/tolune 4/1 was added to the hexane phase, which was then recycled again into the reactor with new portions of 30 ml aldehydes and 300 mg BINAP-2. After heating for half an hour at 100 °C under 30 bar of CO/H₂ =1/1, the mixture was cooled back and separated under N₂. ICP showed 144 ppm Rh content in the obtained residual hexane phase (48 ml). The separated aldehyde extract was united with the previous aldehyde extracts obtaining 134 ml (131,4g) united aldehyde phases, which showed 62 ppm Rh-content. This corresponded to a total of 62 % Rh extraction efficiency of the last three aldehyde extraction steps. The total efficiency of all extraction steps including also the aqueous and the hexane extractions for the separation and recovery of Rh was about 50.7 %. GC analysis was carried out from the united aldehyde extract as well as from the residual hexane phase obtained after repeated aldehyde extractions and from the residual polar organic phase obtained above after repeated hexane extractions. GC analysis showed similarly low high-boiler concentrations in the united aldehyde extract and in the residual hexane phase. However, the residual polar organic phase showed more than 10 times higher high-boiler concentration than that present in the former fragments. Example 4 demonstrates that method 2 using an oxidative treatment is an efficient process for the separation of phosphite degradation products and high-boiler compounds from Rh, as well as for the regeneration of the Rh-bisphosphite based catalyst. Furthermore, Example 4 also demonstrates the repeated use of extraction steps, which can increase the efficiency of Rh-recovery.

### Example 5

A 41 ml amount (41.6 g) of test solution D was treated with 40 ml of an aqueous solution containing 6.5 w% of sodium hypochlorite with gentle stirring at room temperature for 2 hours under air. During and after the treatment a two-phase mixture was present. About 40 ml of organic phase was separated then, which went on the top during separation. A ³¹P NMR spectrum taken from the organic phase showed mostly the presence of the dioxide derivative of fat-BINAP-2 (80 %) beside two other unidenfied minor oxide peaks. From the separated aqueous bleach phase ICP showed 60 ppm of Rh and 18 ppm of P present, which corresponds to 13.7 % Rh- and 3.5 % P- loss to the aqueous phase. Then 20 ml of acetonitrile was added to the separated organic phase and the obtained polar mixture was extracted with 3x40 ml portions of hexane by using a separation funnel under air. During the multiple extraction the amount of polar organic phase gradually decreased from about 57 to 52 g. ICP analysis from all the three hexane extracts showed less than 10 ppm Rh-content, which corresponded to less than 1.2 % Rh-loss per extraction step. ICP analysis from the hexane extracts showed 234, 173 and 124 ppm P in the first, second and third hexane extract, respectively.
This corresponded to 25, 19.5 and 13.7 % removal of the initial P-content by the first, second and third extraction, respectively. In total, by the three hexane extractions less than 3.9 % of the initial Rh-content was lost, while 58.7 % of the initial P-content was removed. An amount of 50 ml from the obtained residual organic phase (which contained 325 ppm Rh and 179 ppm P) was then degassed by using 6 cycles of vacuum and readmission of N₂. Next, 50 ml of degassed hexane was added to the organic phase followed by 525 mg of fat-BINAP-2 (approximately 2.5 mmol bisphosphite per mol Rh) and 35 ml of hexane. The obtained two-phase mixture was autoclaved for 45 minutes at 100 °C under 30 bar CO/H₂ =1/1. After cooling back and venting under N₂, the obtained two-phase mixture was removed from the autoclave and approximately 45 ml of almost colorless hexane phase was then separated from the top. ³¹P NMR spectrum taken from the hexane phase showed only the presence of HRh(CO)₂(Fat-BINAP-2) as Rh complex, which is thought to be a catalytically active species. About 55 % of Fat-BINAP-2 was present as unchanged, free ligand and only 5% of fat-BINAP-2 was degraded to oxide. The separated polar organic phase was washed with a new portion of hexane at room temperature under N₂. The hexane washing was united with the above separated hexane phase. ICP analysis from this fragment (65.9 g) showed 238 ppm Rh-content, while in the residual polar phase less than 10 ppm was detected. This corresponded to 96.7 % Rh-extraction efficiency for the extraction of Rh from the polar phase to hexane by using fat-BINAP-2 ligand. The total efficiency of all extraction steps including also the aqueous and the hexane extractions for the separation and recovery of Rh was about 79.6 %. Example 5 demonstrates that method 3 and 4 via an oxidative treatment is an efficient process for the separation of lipophilic phosphite degradation products. Example 5 also demonstrates that method 3 is an efficient process for the regeneration of the Rh-lipophilic bisphosphite based catalyst.

## Claims

1. A process for the recovery of rhodium from an organic reaction mixture containing rhodium/phosphite ligand complex, phosphite degradation products and high-boiler organic compounds, wherein
A) the organic reaction mixture is subjected to an aqueous oxidative and/or acidic treatment, resulting in an aqueous-polar organic two-phase mixture,
B) the aqueous phase is removed,
C) solution of lipophilic phosphine and/or lipophilic phosphite in a non-polar solvent is added to the organic phase, which solvent is immiscible with the polar organic phase, resulting in a two-phase mixture containing a non-polar organic phase rich in rhodium and lipophilic phosphine and/or lipophilic phosphite and a polar organic phase rich in phosphite degradation products and high-boiler organic compounds, or the polar organic phase is extracted with an immiscible non-polar organic solvent, resulting in a two-phase mixture containing a non-polar organic phase rich in phosphite degradation products and a polar organic phase rich in rhodium,
D) the non-polar organic phase is separated from the polar organic phase.

2. A process according to claim 1 for the recovery of rhodium from an organic reaction mixture containing rhodium/non-lipophilic phosphite ligand complex, non-lipophilic phosphite ligand degradation products and high-boiler organic compounds, wherein
A) the organic reaction mixture is subjected to an aqueous oxidative and/or acidic treatment, resulting in an aqueous-polar organic two-phase mixture,
B) the aqueous phase is removed,
C) a solution of lipophilic phosphite in a non-polar solvent is added to the organic phase, which solvent is immiscible with the polar organic phase, resulting in a two-phase mixture containing a non-polar organic phase rich in rhodium and lipophilic phosphine and/or lipophilic phosphite and a polar organic phase rich in phosphite degradation products and high-boiler organic compounds,
D) the non-polar organic phase is separated from the polar organic phase.

3. A process according to claim 1 for the recovery of rhodium from an organic reaction mixture containing rhodium/lipophilic phosphite ligand complex, lipophilic phosphite ligand degradation products and high-boiler organic compounds, wherein
A) the organic reaction mixture is subjected to an aqueous oxidative and/or acidic treatment, resulting in an aqueous-polar organic two-phase mixture,
B) the aqueous phase is removed,
C) the polar organic phase is extracted with an immiscible non-polar organic solvent, resulting in a two-phase mixture containing a non-polar organic phase rich in lipophilic phosphite degradation products and a polar organic phase rich in rhodium,
D) the non-polar organic phase is separated from the polar organic phase.

4. A process for the recovery of rhodium from an organic reaction mixture containing rhodium/non-lipophilic phosphite ligand complex, non-lipophilic phosphite ligand degradation products and high-boiler organic compounds, regeneration and recycling of the separated Rh-containing fraction as Rh/non-lipophilic phosphite ligand complex, wherein
A) the organic reaction mixture is subjected to an aqueous oxidative and/or acidic treatment, resulting in an aqueous-polar organic two-phase mixture,
B) the aqueous phase is removed,
C) a solution of lipophilic phosphine and/or lipophilic phosphite in a non-polar solvent is added to the organic phase, which solvent is immiscible with the polar organic phase, resulting in a non-polar organic and polar organic two-phase mixture,
D) the non-polar organic phase rich in rhodium and lipophilic phosphine and/or lipophilic phosphite is separated from the polar organic phase rich in phosphite degradation products and high-boiler organic compounds,
E) a non-lipophilic phosphite ligand is added to the non-polar organic phase in a polar solution which is immiscible with the non-polar organic phase, resulting in a non-polar organic and polar two-phase mixture,
F) the non-polar organic phase rich in lipophilic phosphine and/or lipophilic phosphite is separated from the polar organic phase rich in rhodium and non-lipophilic phosphite ligand complex and
G) the polar organic phase is recycled to the reactor.

5. A process for the recovery of rhodium from an organic reaction mixture containing rhodium/lipophilic phosphite ligand complex, lipophilic phosphite ligand degradation products and high-boiler organic compounds, regeneration and recycling of the separated Rh-containing fraction as Rh/lipophilic phosphite ligand complex, wherein
A) the organic reaction mixture is subjected to an aqueous oxidative and/or acidic treatment, resulting in an aqueous-polar organic two-phase mixture,
B) the aqueous phase is removed,
C) the polar organic phase is extracted with an immiscible non-polar organic solvent, resulting in a two-phase mixture containing a non-polar organic phase rich in lipophilic phosphite degradation products and a polar organic phase rich in rhodium,
D) the non-polar organic phase is separated from the polar organic phase,
E) a solution of lipophilic phosphite in a non-polar solvent is added to the polar organic phase, which solvent is immiscible with the polar organic phase, resulting in a polar and non-polar organic two-phase mixture,
F) the non-polar organic phase containing rhodium/lipophilic phosphite ligand complex is separated from the polar organic phase, and
G) the non-polar organic phase is recycled to the reactor.

6. Process according to any one of claims 1-5, wherein the non-polar/polar organic two-phase mixture is subjected to a reductive treatment.

7. Process according to claim 6 and 2, 4 or 5, wherein the reductive treatment is carried out after the addition of lipohilic ligand .

8. Process according to claim 6 and 3 or 4, wherein the reductive treatment is carried out after the addition of non-lipophilic ligand.

9. Process according to claims 6 or 8, wherein the reductive treatment is performed using syngas.

10. Process according to any one of claims 1-9, wherein the polar organic phase obtained in step A is neutralized.

11. Process according to any one of claims 1-10, wherein a polar solvent is added to the polar organic phases.

12. Process according to anyone of claims 1-11, wherein step A is performed by treating the mixture with an oxidizing agent.

13. Process according to claim 12, wherein the oxidizing agent is hypochlorite in aqueous solution.

14. Process according to anyone of claims 1-11, wherein step A is performed by treating the mixture with an aqueous solution of a strong non-coordinating mineral acid.

15. Process according to claim 14, wherein the strong non-coordinating mineral acid is sulfuric acid.

16. Process according to claim 4, wherein a lipophilic monodentate phosphite or phophine ligand is used containing at least one C₉-C₄₀ aliphatic group positioned as a tail extending away from the backbone.

17. Process according to claim 2,or 5, wherein a lipophilic bidentate phosphite ligand is used containing at least one C₉-C₄₀ aliphatic group positioned on the backbone or connecting structures of the bidentate ligand.

18. Process according to anyone of claims 1-2,4 or 5, wherein the lipophilic phosphite ligand is dissolved in an extraction solvent.

19. Process according to claim 18, wherein the extraction solvent is an apolar extraction solvent.

20. Process according to claim 4, wherein the added non-lipophilic bisphosphite ligand is the same as the non-lipophilic bisphosphite ligand present in the organic mixture to be treated.

21. Process according to claim 2 or 5, wherein the added lipophilic bisphosphite ligand differs only in the length of aliphatic group positioned at the backbone or connecting structure from the non-lipophilic bisphosphite ligand, which is present in the organic mixture before the treatment.

22. Process according to claim 3 or 4, wherein the added non-lipophilic bisphosphite ligand differs only in the length of aliphatic group positioned at the backbone or connecting structure from the lipophilic bisphosphite ligand, which is present in the organic mixture before the treatment.

23. Process according to any one of claims 1-22, wherein the P to Rh molar ratio in the organic mixture to be treated is more than 20:1.

24. Process according to any one of claims 1-23, wherein triphenylphosphine or tris(orhtotolyl)phosphine is added to the organic mixture to bring the P to Rh molar ratio above 20:1 when this is less in the organic mixture to be treated.

25. Process according to any one of claims 1-11, wherein one or more steps are repeated.

26. Process according to any one of claims 1-11 or 25, wherein one or more methods are combined.

27. Process according to any one of claims 1-10, wherein the organic mixture to be treated is derived from a hydroformylation, hydrogenation, carbonylation or carboxylation process.
